# EUROPEAN PATENT APPLICATION

(11) **EP 3 815 701 A1**
(43) Date of publication of application: **05.05.2021**
(21) Application number: 19847144.3
(22) Date of filing: 08.08.2019
(51) Int. Cl.: A61K 35/744, A23L 33/135, A61K 8/99, A61K 35/74, A61P 11/02, A61P 11/06, A61P 31/00, A61P 31/12, A61P 37/08, A61P 43/00, A61Q 19/00

(54) **COMPOSITION FOR SUPPRESSING EXPRESSION OF FC?RI, AND FC?RI EXPRESSION SUPPRESSION METHOD**

(30) Priority: 09.08.2018 JP 2018150152
(71) Applicant: Kikkoman Corporation, Noda-shi, Chiba 278-8601 (JP); National University Corporation Chiba University, Chiba-shi, Chiba 263-8522 (JP)
(72) Inventor: ISHIKAWA, Yuki, Noda-shi, Chiba 278-8601 (JP); KAWASHIMA, Tadaomi, Noda-shi, Chiba 278-8601 (JP); ADACHI, Masaki, Noda-shi, Chiba 278-8601 (JP); SHIMOJO, Naoki, Chiba-shi, Chiba 260-8670 (JP)
(74) Representative: Schulz Junghans Patentanwälte PartGmbB
(86) International application number: PCT/JP2019/031341
(87) International publication number: WO 2020/032166

(57) **Abstract**

The purpose of the present invention is to provide a composition containing, as an active ingredient, a substance having rich history of use in foods or an ingestion experience in human and having an effect of inhibiting FcεRI expression on a pDC. This purpose can be achieved by a composition for activating plasmacytoid dendritic cell comprising: at least one active ingredient selected from the group consisting of a bacterial cell, a bacterial component, and a culture of *Pediococcus* lactic acid bacteria, and a product obtained by treating any one of them, and having an effect of inhibiting FcεRI expression on a plasmacytoid dendritic cell.

## Description

### Technical Field

The present invention relates to a composition for inhibiting FcεRI expression and a method of inhibiting FcεRI expression.

### Background Art

In recent years, an increasing number of people are suffering from allergic diseases classified as type I allergies, such as bronchial asthma, allergic rhinitis, hay fever, atopic dermatitis, anaphylactic reactions, urticaria, and food allergies.

In the type I allergy, when allergens (antigens) such as pollen and molds invade the body, IgE corresponding to the allergen is produced by B cell. The produced IgE binds to a high-affinity IgE receptor (FcεRI) expressed on the surface of a mast cell and a basophil (sensitization).

In such the sensitized state, the allergen that re-invades the body binds to two or more IgE bound to different FcεRI and crosslinks two or more FcεRI via IgE (cross-linking reaction). At this time, the mast cell and basophil are activated to cause degranulation, and chemical mediators such as histamine and serotonin are released. As a result, allergic symptoms appear due to vasodilation, hyperpermeability, leukocyte migration, and the like.

Among dendritic cells, a plasmacytoid dendritic cell (pDC) has FcεRI on the cell surface. Invasion of the allergen into the body can cause a cross-linking reaction at the pDC. The pDC is different from a myeloid dendritic cell (mDC) having a dendritic morphology. The pDc is present in the peripheral blood and exhibit traits similar to a plasma cell. The pDC differentiates into dendritic cell-like when stimulated by taking up foreign substances such as viruses.

When the pDC responds to the viruses as the foreign substances, a large amount of type I IFN such as interferon-a (IFN-α) is produced. An antiviral effect of type I IFN inhibits viral growths or replications, and activates an innate immunity.

However, when the cross-linking reaction between IgE antibodies occur via the allergens at the pDC surface, an expression of TLR (toll like receptor), which is cell surface type and cell membrane-penetrating type recognition receptors for the pDC to detect foreign substances, and an expression of interferon regulatory factor (IRF) are suppressed. As a result, the production of IFN-α according to the pDC is suppressed. (See Non-Patent Document 1. The entire document is incorporated as disclosed herein.)

Accordingly, in order to prevent the cross-linking reaction in the pDC in anticipation of the antiviral effect on the pDC, it is considered to suppress the FcεRI expression which has a high affinity for the IgE antibody. For example, Patent Documents 1 to 3 described below (these entire documents are incorporated as disclosed herein) describe methods of screening DNA that regulates FcεRI gene transcriptions and substances that repress FcεRI gene expressions using DNA as an indicator.

### Citation List

### Patent Document

Patent Document 1: JP5131688
Patent Document 2: WO2004/108929
Patent Document 3: WO1999/46393

### Non-Patent Document

Non-Patent Document 1: Michelle Ann Gill, J Allergy Clin Immunol, April 2012, Vol.129, Number 4, pp.889-901

### Summary of Invention

### Problems to be solved by the invention

According to the Patent Documents 1 to 3, it is possible to screen for inhibiting substances of FcεRI gene expression. However, there is no disclosure in Patent Documents 1 to 3 that the substances were obtained by screening. Actually, it is difficult to obtain the inhibiting substances of FcεRI gene expression according to the screening methods described in Patent Documents 1 to 3.

Little is known of substances having rich history of use in foods or an ingestion experience that can be used in humans and having an effect of inhibiting FcεRI expression on the pDC.

An object to be solved by the present invention is to provide a composition containing, as an active ingredient, a substance having rich history of use in foods or an ingestion experience in human and having an effect of inhibiting the expression of FcεRI present on a pDC surface.

### Means for solving the problem

As a result of extensive research to solve the above-described object, the inventors of the present invention have found that *Pediococcus* lactic acid bacteria such as *Pediococcus acidilactici* and *P. pentosaceus* exhibits the effect of inhibiting FcεRI expression on the pDC. Meanwhile, they have found that there are lactic acid bacteria that promote the production of IFN-α based on the pDC but do not exhibit the effect of inhibiting FcεRI expression. It is a very surprising fact found for the first time by the inventors of the present invention that the effect of inhibiting FcεRI expression on the pDC does not correlate with the effect of promoting IFN-α production.

Based on these facts, the inventors of the present invention have succeeded in creating a composition containing, as an active ingredient, a bacterial cell, a bacterial component, and a culture of *Pediococcus* lactic acid bacteria, and a product obtained by treating any one of them and having the effect of inhibiting FcεRI expression on the pDC. The present invention has been completed based on these findings and successful examples.

According to one embodiment of the present invention, compositions, methods and uses of the embodiment described below are provided.
[1] A composition for activating plasmacytoid dendritic cell, containing at least one active ingredient selected from the group consisting of a bacterial cell, a bacterial component, and a culture of *Pediococcus* lactic acid bacteria, and a product obtained by treating any one of them, and having an effect of inhibiting FcεRI expression on a plasmacytoid dendritic cell.
[2] The composition according to [1], wherein the composition is a composition having an effect of promoting interferon-a production on the plasmacytoid dendritic cell.
[3] The composition according to [1] or [2], wherein *Pediococcus* lactic acid bacteria is at least one *Pediococcus* lactic acid bacteria selected from the group consisting of *Pediococcus acidilactici* and *P. pentosaceus.*
[4] The composition according to any one of [1] to [3], wherein the composition has a stronger effect of inhibiting FcεRI expression on a plasmacytoid dendritic cell than that when using *Lactobacillus pentosus* strain K133 in the same amount as the active ingredient.
[5] The composition according to any one of [1] to [4], wherein the composition is at least one anti-desease composition selected from the group consisting of an antiviral composition, a composition for anti-infectious diseases, a composition for anti-allergy, a composition for anti-hay fever, and a composition for anti-asthma.
[6] A composition for foods and drinks which comprise the composition according to any one of [1] to [5].
[7] A method of screening lactic acid bacteria for activating plasmacytoid dendritic cell, including: incubating a test solution comprising at least one ingredient selected from the group consisting of a bacterial cell, a bacterial component, and a culture of lactic acid bacteria, and a product obtained by treating any one of them and a plasmacytoid dendritic cell, and incubating a control solution comprising a plasmacytoid dendritic cell without the ingredient, measuring FcεRI expression levels of the plasmacytoid dendritic cell in the test solution and the control solution after the incubating, and
   determining if the ingredient is an active ingredient that activates the plasmacytoid dendritic cell when the measured FcεRI expression level of the plasmacytoid dendritic cell in the test solution is less than the measured FcεRI expression level of the plasmacytoid dendritic cell in the control solution.
[8] A method of screening lactic acid bacteria for activating plasmacytoid dendritic cell, including: incubating a test solution comprising at least one ingredient selected from the group consisting of a bacterial cell, a bacterial component, and a culture of lactic acid bacteria, and a product obtained by treating any one of them and a plasmacytoid dendritic cell, and incubating a control solution comprising a plasmacytoid dendritic cell without the ingredient,
   measuring interferon-a production amounts and FcεRI expression levels of the plasmacytoid dendritic cell in the test solution and the control solution after the incubating, and determining if the ingredient is an active ingredient that activates the plasmacytoid dendritic cell when the measured interferon-a production amount in the test solution is more than the interferon-a production amount in the control solution and the measured FcεRI expression level of the plasmacytoid dendritic cell in the test solution is less than the measured FcεRI expression level of the plasmacytoid dendritic cell in the control solution.
[9] A method of inhibiting FcεRI expression on plasmacytoid dendritic cell, including: using at least one active ingredient selected from the group consisting of a bacterial cell, a bacterial component, and a culture of *Pediococcus* lactic acid bacteria, and a product obtained by treating any one of them.
[10] Use of at least one active ingredient selected from the group consisting of a bacterial cell, a bacterial component, and a culture of *Pediococcus* lactic acid bacteria, and a product obtained by treating any one of them for manufacturing a composition for inhibiting FcεRI expression of a plasmacytoid dendritic cell.
[11] Use of at least one active ingredient selected from the group consisting of a bacterial cell, a bacterial component, and a culture of *Pediococcus* lactic acid bacteria, and a product obtained by treating any one of them for inhibiting FcεRI expression of a plasmacytoid dendritic cell.

### Effect of the Invention

The composition according to one embodiment of the present invention has the effect of inhibiting FcεRI expression on the pDC, and in relation to the effect, it can be expected that the composition further exhibits antiviral effects, anti-infective effects, anti-allergy effects, anti-hay fever effects, anti-asthma effects, and the like.

The active ingredient used in the composition according to one embodiment of the present invention is an ingredient which is proven for use as additives and the like in foods and drinks. Therefore, the composition according to one embodiment of the present invention is highly safe and is useful as foods and drinks, medicines, quasi-drugs, cosmetics, and the like having antiviral effects, anti-infective effects, anti-allergy effects, anti-hay fever effects, and anti-asthma effects. It can be expected that the composition is provided in an oral or parenteral form.

### Brief Description of Drawings

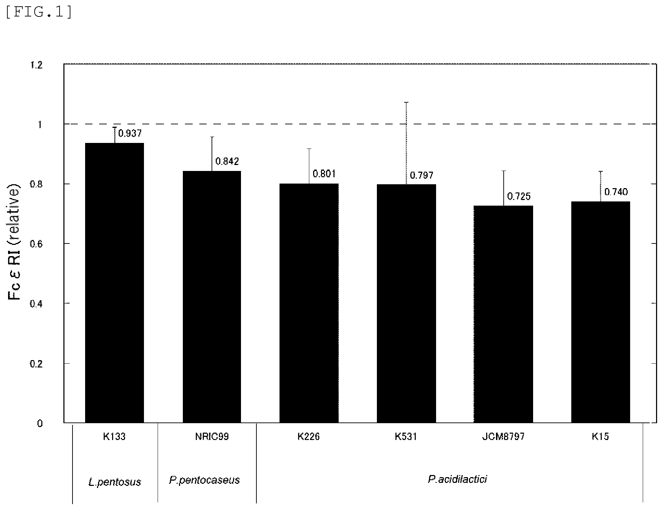

[Fig.1] Fig.1 is a graph showing measurement results of relative FcεRI expression levels on the pDC in response to *Lactobacillus pentosus* strain K133 ("K133"), *P. pentosaceus* strain NRIC99 ("NRIC99"), and *Pediococcus acidilactici* strains K226, K531, JCM8797, and K15 ("K226", "K531", "JCM8797", and "K15"), which is described in examples.

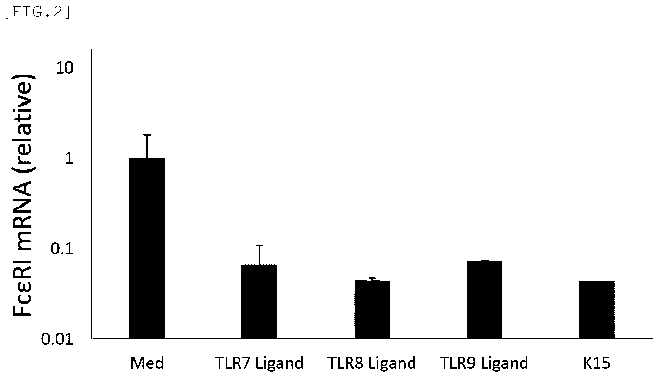

[Fig.2] Fig.2 is a graph showing results of measuring an amount (relative amount) of FcεRI mRNA produced by the pDC cultured with TLR7 ligand, TLR8 ligand, TLR9 ligand, and *Pediococcus acidilactici* strain K15 ("K15"), which is described in examples. In addition, "Med" shows a control (the result of measuring without the lactic acid bacteria).

### Description of Embodiments

The present invention will now be described in detail. The technical scope of the present invention is not limited to the matter in this section; rather, the present invention may take various other forms to the extent that its objectives are achieved.

The composition according to one embodiment of the present invention contains at least one active ingredient (hereinafter, will simply be referred to as "active ingredient") selected fro m the group consisting of a bacterial cell, a bacterial componen t, and a culture of *Pediococcus* lactic acid bacteria, and a prod uct obtained by treating any one of them.

The composition according to one embodiment of the present invention may have an effect of inhibiting FcεRI expression on a plasmacytoid dendritic cell or an effect of inhibiting FcεRI e xpression on a plasmacytoid dendritic cell and an effect of prom oting interferon-a production on a plasmacytoid dendritic cell, depending on the active ingredient included. In the specificati on, these effects may be collectively referred to as "active eff ect". The composition according to one embodiment of the presen t invention may typically take the embodiment of a composition f or activating a plasmacytoid dendritic cell by having the active effect. Another embodiment according to the present invention is a method of inhibiting FcεRI expression on a plasmacytoid den dritic cell using the active ingredient.

Unless otherwise specified, each term in the specification is used in the sense normally used by those skilled in the art a nd should not be construed as having an unduly restrictive meani ng.

For example, the term "composition" is not specifically li mited and the meaning usually used. The composition is an objec t consisting of a combination of two or more substances. Specif ic examples thereof include a combination of an active ingredien t and another substance, and a combination of two or more active ingredients. More specifically, solid compositions and liquid compositions in which one or more active ingredients and one or more solid carriers or solvents are combined can be mentioned.

The phrase "effect of inhibiting expression" with the plasmacytoid dendritic cell refers to at least one effect selected from the group consisting of inhibiting expression level of genes, decreasing transcription level of gene products (proteins), and activating plasmacytoid dendritic cells so as to inhibit the gene products.

The phrase "effect of promoting production" with the plasmacytoid dendritic cell refers to at least one effect selected from the group consisting of inhibiting expression level of genes, increasing transcription level of gene products, and activating plasmacytoid dendritic cells so as to produce the gene products.

The phrase "activating plasmacytoid dendritic cell" means that the active ingredient keeps the plasmacytoid dendritic cell in a state of exerting the active effect.

The term "and/or" means any one, or any combination or all of two or more, of the multiple related items listed.

FcεRI (high-affinity IgE receptor) is not particularly limited and having a commonly known meaning. It can be said to be a high-affinity receptor for the Fc portion of IgE on the surface of plasmacytoid dendritic cell and mast cell. FcεRI has three types of subunits, α chain, β chain and γ chain. It is known to be expressed on the cell surface in the forms of a tetramer structure of αβγ₂ and a trimeric structure of αγ₂ in mast cells and basophils, and in the form of a trimeric structure of αγ₂ in dendritic cells such as plasmacytoid dendritic cells, monocytes, and eosinophils.

When an antigen binds to two or more sets of IgE (FcεRI-IgE) bound to the α chain of FcεRI, FcεRI is crosslinked by the antigen. Such cross-linking results in intracellular signaling through the γ and β chains to activate the plasmacytoid dendritic cells and/or mast cells.

Interferon (IFN)-α is a type I interferon and has an antiviral effect that suppresses virus growth and/or replication. In addition, IFN-α has a growth inhibitory effect on virus-infected cells, an activating effect on cells involved in natural immunity such as macrophages and natural killer cells (NK cells), an antitumor effect, and a growth inhibitory effect of intracellular parasites other than viruses. IFN-α is clinically applied as a therapeutic agent for viral hepatitis.

A plasmacytoid dendritic cell (pDC) is present in the peripheral blood and exhibits traits similar to plasmacytoid cells and differentiate into dendritic cells when stimulated by taking up foreign substances. The active ingredient acts on the pDC to cause the active effect.

A method of evaluating the effect of inhibiting FcεRI expression is not particularly limited, and for example, it can be evaluated by measuring the FcεRI expression level expressed by the pDC, as described in the examples below.

An example of the method of evaluating the effect of inhibiting FcεRI expression includes, but is not limited, the method including: co-culturing the pDC derived from each subject with the composition according to one embodiment of the present invention, and then labeling FcεRI on the surface of the cells recovered or the culture supernatant after the culturing as a test sample, with a commercially available labeled anti-FcεRI antibody, subsequently measuring the labeling intensity of the labeled FcεRI with such as flow cytometry, and/or measuring the FcεRI expression level on the pDC derived from each subject using a commercially kit for measuring an amount of FcεRI mRNA in the cells or culture supernatant. The condition of co-culturing is not particularly limited, and for example, conditions that do not inhibit the FcεRI expression and do not kill the pDC can be mentioned, and it is preferable that the medium, temperature, CO₂ concentration, pH, etc. suitable for the maintenance or growth of pDC are adopted as the culture condition.

The number of subjects in the method of evaluating the effect of inhibiting FcεRI expression is preferably 2 or more, and more preferably 3 or more, in consideration of individual differences. The effect of inhibiting FcεRI expression is evaluated by averaging relative values calculated from the FcεRI expression level on the pDC derived from each subject based on a control (a system without the composition according to one embodiment of the present invention) between subjects. For example, D_{A}, D_{B}, ··· , and Dₓ, which are the FcεRI expression levels on the pDC derived from each subject A, subject B, ···, and subject X, are measured, then R_{A}, R_{B}, ···, and Rₓ, which are the relative values, are calculated from D_{A}, D_{B}, ···, and Dₓ based on the control, and then the average relative value AR_{AB···X} calculated from R_{A}, R_{B}, ···, and Rₓ to evaluate.

A degree of the effect of inhibiting FcεRI expression is not particularly limited, for example, but is such that the FcεRI expression level on the pDC is evaluated to be less than that when the composition according to one embodiment of the present invention is not used (control). However, it is preferable that the degree of effect of inhibiting FcεRI expression is a degree evaluated to be less than that of the control at any of the FcεRI expression levels on the pDC derived from each subject (in the above example, 1> R_{A}, 1> R_{B}, ···, and 1> R_{X}).

As described in examples below, it is more preferable that the effect of inhibiting FcεRI expression in the composition according to one embodiment of the present invention is a degree evaluated to be less than the FcεRI expression level on the pDC when using *Lactobacillus pentosus* strain K133 in the same amount as the active ingredient because the effect of inhibiting FcεRI expression possessed by *Pediococcus* lactic acid bacteria tends to be stronger than that of *Lactobacillus pentosus* strain K133. In the more preferable embodiment, in the above example, it is the degree that the average relative value AR_{AB···X} obtained with the composition according to one embodiment of the present invention is less than the average relative value AR_{AB···X} obtained with *Lactobacillus pentosus* strain K133 in the same amount as the active ingredient.

If *Pediococcus* lactic acid bacteria is *Pediococcus acidilactici,* the degree of effect of inhibiting FcεRI expression is preferably that the FcεRI expression level on the pDC is evaluated to be less than that when using *Pediococcus pentosaceus* strain NRIC99 in the same amount as the active ingredient.

The degree of effect of inhibiting FcεRI expression is, for example, such that the average relative value is less than or equal to 0.93, which is calculated from the FcεRI expression level in cells recovered 24 hours after co-culturing 1 x 10⁷ cells of *Pediococcus* lactic acid bacteria and 2.5 x 10⁴ cells of pDC in 200 µl. It is preferably 0.90 or less, more preferably 0.85 or less, further preferably 0.83 or less, and even more preferably 0.81 or less. The lower limit is not limited, but is typically 0.10 or more.

A method of evaluating the effect of promoting IFN-α production is not particularly limited, and for example, it can be evaluated by measuring the amount of IFN-α produced by the pDC and/or the expression level of IFN-α gene expressed by the pDC, as described in the examples below.

An example of the method of evaluating the effect of promoting IFN-α production includes, but is not limited, the method including: co-culturing the pDC and the composition according to one embodiment of the present invention, and then measuring the IFN-α concentration using a commercially kit the culture supernatant recovered after culturing as a test sample. The condition of co-culturing is not particularly limited, for example, conditions that do not deactivate the IFN-α and do not kill the pDC can be mentioned, and it is preferable that the medium, temperature, CO₂ concentration, and pH, etc. suitable for the maintenance or growth of the pDC are adopted as the culture condition.

A degree of the effect of promoting IFN-α production is not particularly limited, but is preferable such that the IFN-α production amount on the pDC is more than that when the composition according to one embodiment of the present invention is not used. If *Pediococcus* lactic acid bacteria is *Pediococcus acidilactici,* the degree of effect of promoting IFN-α production is preferably such that the IFN-α production amount on the pDC is more than that when *Pediococcus pentosaceus* strain NRIC99 in the same amount as the active ingredient is used.

A degree of the active effect is, for example, such that a concentration of IFN-α (average value among subjects) in the supernatant recovered 24 hours after co-culturing 1 × 10⁷ cells of *Pediococcus* lactic acid bacteria and 2.5 × 10⁴ cells of pDC in 200 µl is preferably 10 pg/ml or more, more preferably 100 pg/ml or more. The upper limit is not limited, but is typically 10,000 pg/ml or less.

It is expected that the composition according to one embod iment of the present invention which has the active effect exhib its antiviral effects, anti-infective effects, anti-allergy eff ects, anti-hay fever effects, anti-asthma effects, and the like.

Therefore, the composition according to one embodiment of the present invention can adopt some embodiments such as antiviral c ompositions, compositions for anti-infectious diseases, composi tions for anti-allergy, compositions for anti-hay fever, and com positions for anti-asthma.

An antiviral effect, an anti-infective effect, an anti-all ergy effect, an anti-hay fever effect, and an anti-asthma effect exhibited by the composition according to one embodiment of the present invention mean, for example, in the case of anti-infect ive effects, to improve, alleviate or treat the present or futur e symptoms of the viral disease, or to suppress, delay or preven t to get the condition considered as suffering from the viral di sease, in an individual with intake of the composition of one em bodyment of the present invention. In terms of anti-asthma effec ts, it is known that omalizumab, which is an anti-IgE antibody, improves asthma symptoms, and it is considered that a part of th is mechanism is due to the effect of inhibiting FcεRI expression • (For example, see Non-Patent Document 1, William W. Busse, et al., N Engl J Med, March 2011, Vol. 17, Number 364, pp. 1005-101 5, Calman Prussin, et al., J Allergy Clin Immunol, December 2003 , Vol. 112, Number 6, pp1147-1154; the entire document is incorp orated as disclosed herein.)

Non-Patent Document 1 describes that the presence of aller gens causes a cross-linking reaction at FcεRI in the pDC, result ing in a decrease in the IFN-α production amount. According to the document of Michelle et al (Michelle A. Gill et al., J Immun ol. 2010 June 1; 184 (11): 5999-6006; the entire document is inc orporated as disclosed herein.), it is described that the FcεRI expression level in the pDC is higher in asthmatic patients than that in healthy subjects, and that the IFN-α production amount based on the pDC is less in asthmatic patients with influenza th an that in healthy subjects with influenza. Considering the int egration of these descriptions, the composition according to one embodiment of the present invention may exert a high degree of antiviral and anti-infective effects in the asthmatic patients.

The active ingredient of the composition according to one embodiment of the present invention is at least one active ingredient selected from the group consisting of a bacterial cell, a bacterial component, and a culture of *Pediococcus* lactic acid bacteria, and a product obtained by treating any one of them.

As generally known, *Pediococcus* lactic acid bacteria is no t particularly limited as long as lactic acid bacteria belonging to the genus *Pediococcus* which is a coccus that produces gram-p ositive lactic acid. Examples of *Pediococcus* lactic acid bacter ia include *Pediococcus* lactic acid bacteria having rich history of use in foods.

A method of obtaining *Pediococcus* lactic acid bacteria is not particularly limited. For example, *Pediococcus* lactic acid bacteria may be isolated as an isolated strain from lactic acid bacteria-containing substances such as fermented foods such as p ickled products, sake lees, dairy products, and soy sauce *moromi* , and may also be strains distributed from American Type Culture Collection (ATCC), Tokyo University of Agriculture (NRIC), Inst itute of Physical and Chemical Research (JCM), National Institut e of Technology and Evaluation Biological Resource Center (NBRC) , and National Institute of Technology and Evaluation Patent Micr oorganisms Depositary (NITE-IPOD).

Specific examples of *Pediococcus* lactic acid bacteria include, but are not limited to, *Pediococcus acidilactici, Pediococcus pentosaceus, Pediococcus cellicola, Pediococcus claussenii, Pediococcus damnosus, Pediococcus ethanolidinans, Pediococcus inopinatus, Pediococcus parvulus,* and *Pediococcus stilesii.* As *Pediococcus* lactic acid bacteria, one kind thereof can be used or a combination of two or more kinds thereof can be used.

Non-limiting examples of *Pediococcus acidilactici* and *Pediococcus pentosaceus* include *Pediococcus acidilactici* strain K15, *Pediococcus acidilactici* strain K226, *Pediococcus acidilactici* strain K531, *Pediococcus acidilactici* strain NRIC0124, *Pediococcus acidilactici* strain JCM8797, *Pediococcus pentosaceus* strain OS (NITE P-354), *Pediococcus pentosaceus* strain NRIC1915, *Pediococcus pentosaceus* strain NRIC99, *Pediococcus pentosaceus* strain NRIC122, *Pediococcus pentosaceus* strain JCM2024, and *Pediococcus pentosaceus* strain ATCC33314.

Among *Pediococcus* lactic acid bacteria, *Pediococcus acidilactici* and *Pediococcus pentosaceus* are preferable because they have a large active effect and abundant eating experience. *Pediococcus acidilactici* is more preferable because it has a greater active effect, and *Pediococcus acidilactici* strain K15, *Pediococcus acidilactici* strain K226, *Pediococcus acidilactici* strain K531, and *Pediococcus acidilactici* strain JCM8797 are even more preferable.

Hereinafter, the details of composition according to one embodiment of the present invention will be described on the assumption that *Pediococcus acidilactici* is used as *Pediococcus* lactic acid bacteria. However, when using *Pediococcus* lactic acid bacteria other than *Pediococcus acidilactici,* the term *"Pediococcus acidilactici"* can be replaced with *"Pediococcus pentosaceus"* or some other *Pediococcus* lactic acid bacteria.

A method of isolating *Pediococcus acidilactici* is not particularly limited, and examples of the method include a method including appropriately diluting an extract containing lactic acid bacteria-containing substances according to a conventional method and culturing the extract at about 30°C to 40°C using a known lactic acid bacteria culture medium such as MRS medium, M17 medium, and the like. For example, *Pediococcus acidilactici* strains K15, K226, and K531 are lactic acid bacteria strains isolated from bran beds, pickles, and wine grapes as lactic acid bacteria-containing substances respectively, and their optimal temperature is about 37°C to 42°C.

According to the investigation by the inventors of the present invention, *Pediococcus acidilactici* can contain a high concentration of double-stranded RNA by culturing under stress conditions. Examples of *Pediococcus acidilactici* having large amounts of double-stranded RNA include *Pediococcus acidilactici* containing 15,000 ng or more of doublestranded RNA per 1 × 10¹⁰ cfu of bacterial cells and *Pediococcus acidilactici* of which a ratio of double-stranded RNA in total nucleic acids is 3.5% or more per 1 × 10¹⁰ cfu of bacterial cells. The upper limit is not limited, but is typically 200,000 ng or less and 99 % or less.

The term "stress condition" refers to a condition such as high salt concentration, high temperature, low temperature, oligotrophic conditions, eutrophic conditions, low pH, high pH, aerations, and combination thereof, which is the condition deviate from general condition for culturing lactic acid bacteria, and preferably refers to condition in which a doubling time of lactic acid bacteria becomes longer than that of a case where lactic acid bacteria is cultured under a suitable condition.

Specific examples of culturing under the stress condition due to salt include culturing carried out by using a medium containing a salt concentration of preferably 0.5% to 30% (w/v), and more preferably 5% to 15% (w/v). In a medium in which the salt concentration is 0.5% (w/v) or less or 30% (w/v) or more is not preferable because the growth of *Pediococcus acidilactici* may be extremely slow.

Examples of culturing under the stress condition of the high temperature or low temperature include culturing carried out at an optimum temperature of ±1°C to 30°C which are suitable for the growth of *Pediococcus acidilactici,* and preferably at the optimum temperature of ±5°C to 20°C. Specific examples of the culturing include culturing carried out at 10°C to 39°C or 41°C to 60°C in a case where the optimum temperature for *Pediococcus acidilactici* is 40°C.

In addition, examples of culturing under the stress condition of the aeration include culturing carried out under a stress condition that increases the dissolved oxygen concentration by supplying air and stirring. At this time, oxygen gas may be used instead of air, and shaking or other means may be employed in addition to agitation as long as the dissolved oxygen concentration can be increased. An agitation speed can be set appropriately depending on a size of culture tank and the like.

An amount of supplied air (aeration rate) is not particularly limited, but is, for example, about 0.01 VVM to 10 VVM per unit volume, and preferably about 0.1 VVM to 1 VVM. When the aeration rate is smaller than 0.01 VVM, the dissolved oxygen concentration may not be increased. Meanwhile, when the aeration rate is larger than 10 VVM, the lactic acid bacteria may be physically damaged by the large amount of air bubbles, which is not preferable in the cases.

As for other conditions for culturing *Pediococcus acidilactici,* conditions suitable for the growth of *Pediococcus acidilactici* can be used. For example, a medium used can be a medium usually used for culturing *Pediococcus acidilactici.* Examples of the medium include, but are not limited to, MRS medium, M17 medium, and the like. A culture period of time is not particularly limited as long as the growth of *Pediococcus acidilactici* is observed and a sufficient bacterial density can be obtained.

*Pediococcus acidilactici* which have been cultured under the stress conditions are capable of producing more of bacterial components such as double-stranded RNA, compared to a case in which culturing is carried out under conditions with no stress.

*Pediococcus acidilactici* may be *Pediococcus acidilactici* cultured under the stress conditions, may be *Pediococcus acidilactici* cultured under normal conditions, or may be any *Pediococcus acidilactici* cultured under either the stress conditions or the normal conditions. For example, one kind of, or two or more kinds of *Pediococcus acidilactici* cultured under the stress conditions may be combined with one kind of, or two or more kinds of *Pediococcus acidilactici* cultured under the normal conditions.

The active ingredient in the composition according to one embodiment of the present invention may be the bacterial component of the corresponding *Pediococcus acidilactici,* the culture of the corresponding *Pediococcus acidilactici,* and the product obtained by treating any one of the cell, the bacterial component and the culture of the corresponding *Pediococcus acidilactici,* in addition to *Pediococcus acidilactici* themselves, that is, the bacterial cell of the corresponding *Pediococcus acidilactici.*

Examples of the bacterial cell of *Pediococcus acidilactici* include a bacterial cell obtained by removing a medium from a culture of *Pediococcus acidilactici* by using a generally known solid-liquid separation means such as centrifugation.

Examples of the bacterial component of *Pediococcus acidilactici* include a purified or unpurified component which is present within the bacterial cell of *Pediococcus acidilactici,* or which is secreted outside the bacterial cell, and specific examples of the bacterial component include single-stranded RNA and double-stranded RNA, and single-stranded DNA and double-stranded DNA.

Examples of the culture of *Pediococcus acidilactici* include a culture solution itself obtained by culturing *Pediococcus acidilactici.*

Examples of the product obtained by treating any one of a bacterial cell, a bacterial component, and a culture of *Pediococcus acidilactici* include a product obtained by subjecting the bacterial cell, the bacterial component, and the culture of *Pediococcus acidilactici* to generally known methods of treating bacterial cells, bacterial components, and cultures of lactic acid bacteria, such as sterilization and drying.

The bacterial cells of *Pediococcus acidilactici* are obtained by sterilizing the culture of *Pediococcus acidilactici,* removing the medium by using generally known solid-liquid separation means such as ultrafiltration membrane and centrifugation and recovering bacterial cells, and subsequently, washing the obtained bacterial cells with a washing solution such as water, a saline solution, or the like. The bacterial cells obtained as above can be used as an active ingredient either as it stands or in a form of, for example, a dried powder obtained by mixing it with food materials such as dextrin and other sugars and subjecting the bacterial cells to a drying treatment. A method of the drying treatment is not particularly limited, and examples of the method include natural drying, air drying, spray drying, freeze drying, and the like. The active ingredient contained by the composition according to one embodiment of the present invention is preferably a dried powder of *Pediococcus acidilactici* from the viewpoint of storage stability and ease of handling in production.

The active ingredient is not particularly limited as long as the bacterial cell, the bacterial component, and the culture of *Pediococcus acidilactici* and the product obtained by treating any one of them, but is preferably, for example, the bacterial cell, the bacterial component, and the culture all of which have a nucleic acid, respectively and the product obtained by treating any one of them, more preferably, the bacterial cell, the bacterial component, and the culture all of which have RNA, respectively and the product obtained by treating any one of them, even more preferably, the bacterial cell, the bacterial component, and the culture all of which have double-stranded RNA, respectively and the product obtained by treating any one of them.

The bacterial cell, the bacterial component, and the culture of *Pediococcus acidilactici* and the product obtained by treating any one of them can be used alone, or a combination of two or more kinds thereof can be used. For example, RNA, preferably double-stranded RNA, may be isolated and used from the bacterial cell, the bacterial component, and the culture of *Pediococcus acidilactici* and the product obtained by treating any one of them. The method of isolating RNA from the bacterial cell, the bacterial component, and the culture of *Pediococcus acidilactici* and the product obtained by treating any one of them is not particularly limited, and examples of a method for isolating double-stranded RNA from the bacterial cell of *Pediococcus acidilactici* include the method described in Patent No. 5312322.

A method of obtaining the bacterial cell, the bacterial component, or the culture of *Pediococcus acidilactici* and the product obtained by treating any one of them containing double-stranded RNA is not particularly limited. Examples of the method include a method in which a normal MRS medium is inoculated with *Pediococcus acidilactici,* followed by culturing at 35°C to 45°C for 24 to 72 hours, thereby obtaining the bacterial cell, the bacterial component, or the culture of *Pediococcus acidilactici* and the product obtained by treating any one of them containing double-stranded RNA.

Specific examples of the method of isolating double-stranded RNA from the bacterial cell, the bacterial component, or the culture of *Pediococcus acidilactici* and the product obtained by treating any one of them include the following method. That is, the bacterial cell, the bacterial component, and the culture of *Pediococcus acidilactici* containing double-stranded RNA are subjected to a heat and pasteurization treatment at 90°C to 100°C for 5 to 20 minutes, and therefore a heat and pasteurization-treated liquid is obtained. Subsequently, the heat and pasteurization-treated liquid is subjected to the solid-liquid separation means such as centrifugation, and solid contents are recovered. Subsequently, the solid contents are washed and suspended in a buffer solution to obtain a suspension. Subsequently, lysozyme is added to the suspension and warmed at 35°C to 40°C to obtain a lysozyme-treated solution. Subsequently, SDS and Proteinase K are added to the lysozyme-treated solution and warmed at 35°C to 40°C to obtain a proteinase-treated solution. Subsequently, the proteinase-treated solution was subjected to a phenol-chloroform-isoamyl alcohol treatment, and therefore a crude nucleic acid extract is obtained as a supernatant by the solid-liquid separation means such as centrifugation. The crude nucleic acid extract is a mixture of nucleic acids which contains DNA, single-stranded RNA, double-stranded RNA, and the like. Subsequently, by subjecting the crude nucleic acid extract to cellulose column chromatography, purified double-stranded RNA can be obtained. The cellulose column chromatography and the subsequent advanced purification means can be carried out according to the description of Patent No.: 5312322.

General *Pediococcus acidilactici* has been ingested by humans for a long period of time, and thus is highly safe. Therefore, the composition according to one embodiment of the present invention has high practicality, and can be applied in various applications with or without processing.

A method of ingesting the composition according to one embodiment of the present invention is not particularly limited, for example, it can be applied in an oral or parenteral form. Parenteral application includes, but is not limited to, injection and infusion by intradermal, subcutaneous, intravenous, intramuscular administration, and the like; transdermal; inhalation through mucous membranes such as nose, pharynx, and the like.

The ingesting individual of the composition according to one embodiment of the present invention is not particularly limited, and examples thereof include animals, especially mammals. Examples of mammals include humans, dogs, cats, cows, horses, pigs, sheep, and the like, and among these, humans are preferable. The ingesting individual may be a healthy individual, but is preferably an individual for which an anti-disease effect and/or an immunostimulatory effect is desired.

The composition according to one embodiment of the present invention can be contained, for example, in a combination of active ingredient with other components in accordance of an application. As described above, the composition according to one embodiment of the present invention can be contained with other various components in addition to the bacterial cell, the bacterial component, and the culture of *Pediococcus acidilactici* and the product obtained by treating any one of them as long as the object of the present invention can be achieved by the composition.

Other components include, for example, additives used for general food processing and/or medicine processing such as a sugar sweetener, a stabilizer, an emulsifier, a starch, a processed product of starch, a hydrolyzed product of starch, a salt, a flavoring agent, a coloring agent, an acidulant, a flavor ingredient, a nutrient, a fruit juice, animal and vegetable food ingredients such as an egg, an excipient, a bulking agent, a binder, a thickener, a perfume oil, a preserving agent, and a buffering agent. An amount used of the other component is not particularly limited as long as the solution of the object of the present invention is not hindered by the amount, and the amount can be set appropriately.

The composition according to one embodiment of the present invention is not particularly limited as long as it is in a form generally used, and for example, the composition can adopt various forms such as solid form, liquid form, gel form, suspension form, cream form, sheet form, stick form, powder form, granule form, fine granule form, tablet form, rod form, plate form, block form, paste form, capsule form, and caplet form.

In a case where the composition according to one embodiment of the present invention is, for example, a composition for oral ingestion, the composition is preferably an enteric composition capable of delivering the bacterial cell, the bacterial component, and the culture of of *Pediococcus acidilactici* and the product obtained by treating any one of them to the small intestine via esophagus and stomach. The enteric composition is not particularly limited as long as it is in a form that does not dissolve by gastric acids but dissolves in the small intestine, and examples of the enteric composition include a composition in a form of an acid-resistant microcapsule or a liposome.

A contained amount of the active ingredient is not particularly limited as long as it is an amount by which the effect of inhibiting FcεRI expression to the plasmacytoid dendritic cell, preferably the effect of inhibiting FcεRI expression to the plasmacytoid dendritic cell and the effect of promoting interferon-a production to the plasmacytoid dendritic cell can be recognized. In the case of the composition for oral ingestion, the amount thereof is, for example, 0.0001% to 99% by mass, and preferably 0.001% to 90% by mass with respect to an entire composition. In the case of the composition for parenteral ingestion, the amount thereof is, for example, 0.00001% to 99% by mass, and preferably 0.0001% to 90% by mass with respect to an entire composition.

An ingestion dose of the composition according to one embodiment of the present invention is not particularly limited, and can be appropriately set in accordance with characteristics of the ingested individual, such as age, physique, and symptoms; methods of ingesting; and the site of application. An ingestion dose of the composition, for example, the ingestion dose of the composition containing the cells of *Pediococcus acidilactici* as the active ingredient is generally 1 to 1000 mg/60 kg of body weight/day. An ingestion frequency is not particularly limited, but is, for example, once to three times a day, and the ingestion frequency can be appropriately increased or decreased according to the ingestion dose.

The composition according to one embodiment of the present invention can take a form of a composition for foods and drinks, a composition for medicine, and the like, with the composition alone or as a mixture of the compositions. That is, a specific embodiment of the present invention is a composition for foods or drinks, a composition for medicines, a composition for quasi-drugs, a composition for cosmetics, a composition for animal feed, or the like, containing the active ingredient or the composition according to one embodiment of the present invention.

In a case where the composition according to one embodiment of the present invention is a composition for foods or drinks, any form of foods or drinks can be taken as long as the composition can exert at least the active effect after ingestion. Examples of the foods or drinks include beverages such as fruit juices, vegetable juices, fruit and vegetable juices, tea drinks, coffee drinks, sports drinks, soft drinks, and health drinks; dairy products such as yogurt and cheese; processed foods and drinks such as soups, noodles, puddings, and jams; confectionery such as chewing gum, candies, tablets, gummy jellies, chocolates, biscuits, and snacks; frozen confectionery such as ice cream, sherbet, and frozen dessert; and seasonings such as soy sauce and miso.

A specific embodiment of the composition for foods or drinks is, for example, functional foods and drinks, which are foods and drinks having a certain functionality with respect to a living body. Examples of the functional foods and drinks include foods and drinks for specific persons such as foods and drinks for babies, foods and drinks for pregnant women, foods and drinks for aged persons, and the like in addition to health foods and drinks such as foods and drinks for specified health use, foods and drinks with functional claims, nutritional functional foods and drinks, health functional foods and drinks, foods and drinks for a special purpose, nutritional supplement foods and drinks, health supplement foods and drinks, supplements, beauty foods and drinks, and the like. In addition, the functional foods and drinks include health foods and drinks to which a health claim based on the food standards of Codex (Joint FAO/WHO Codex Alimentarius Commission) is applied.

A packaging form of the composition for foods or drinks is not particularly limited and can be appropriately selected depending on an applicable form and the like. For example, packs, bottles, cans, pouches and the like made of coated paper, plastics such as PET and PTP, metals such as aluminum, glass and the like.

In a case where the composition according to one embodiment of the present invention is a composition for medicines, any form of medicines can be taken as long as the composition can exert the effect of inhibiting FcεRI expression to the plasmacytoid dendritic cell, preferably the effect of inhibiting FcεRI expression to the plasmacytoid dendritic cell and the effect of promoting interferon-α production to the plasmacytoid dendritic cell after application and accordingly a desired anti-desease effect.

A dosage form of the composition for medicines is not particularly limited, but examples include injectable (muscle, subcutaneous, intradermal), oral, and nasal preparations. The composition for medicines can be prepared as a vaccine to exert an antiviral effect. When the composition for medicines is in the form of the vaccine, it may be a mixed cocktail vaccine containing a plurality of active ingredients.

A production method of the composition according to one embodiment of the present invention is not particularly limited, but examples include a method of mixing the bacterial cell, the bacterial component, and the culture of of *Pediococcus acidilactici* and the product obtained by treating any one of them, and optionally other components, and forming them into a desired dosage form.

Examples of the production method of the composition according to one embodiment of the present invention include the following method: an MRS medium is inoculated with *Pediococcus acidilactici,* the corresponding Pediococcus acidilactici are cultured at the optimum temperature of ±5°C to 20°C for 12 to 72 hours, and therefore a culture is obtained. Subsequently, the medium is subjected to a heat and pasteurization treatment at 90°C to 100°C for 5 to 20 minutes, and therefore a heat and pasteurization-treated liquid is obtained. The heat and pasteurization-treated liquid is removed by using generally known solid-liquid separation means such as ultrafiltration membrane and centrifugation, and bacterial cells are recovered. Subsequently, the obtained bacterial cells are washed with water, a saline solution, or the like, and therefore the bacterial cells of *Pediococcus acidilactici* are obtained. The bacterial cells obtained as above, a bacterial cell suspension in which the corresponding bacterial cells are suspended in water, a saline solution, or the like, a dry powder obtained by subjecting the corresponding bacterial cells to a drying treatment, or the like can be used as the active ingredient of the composition of the present invention. A method of the drying treatment is not particularly limited, and examples of the method include natural drying, air drying, spray drying, freeze drying, and the like.

The production method of the composition according to one embodiment of the present invention may include various steps and manipulations before, after, or during the above steps as long as it can achieve the objectives of the present invention.

Another embodiment according to the present invention is a method of screening lactic acid bacteria for activating a plasmacytoid dendritic cell, which may be used as the active ingredient of the composition according to one embodiment of the present invention. The method of screening according to one embodiment of the present invention is roughly classified into two specific embodiments.

The first specific embodiment of the method of screening according to the present invention is a method that uses the effect of inhibiting FcεRI expression on the plasmacytoid dendritic cell as an indicator, and includes at least the following steps:
incubating a test solution containing at least one ingredient selected from the group consisting of a bacterial cell, a bacterial component, and a culture of lactic acid bacteria, and a product obtained by treating any one of them and a plasmacytoid dendritic cell, and incubating a control solution containing a plasmacytoid dendritic cell without the ingredient,
measuring FcεRI expression levels of the plasmacytoid dendritic cell in the test solution and the control solution after the incubating, and
determining if the ingredient is an active ingredient that activates the plasmacytoid dendritic cell when the measured FcεRI expression level of the plasmacytoid dendritic cell in the test solution is less than the measured FcεRI expression level of the plasmacytoid dendritic cell in the control solution.

The second specific embodiment of the method of screening according to the present invention is a method that uses the effect of inhibiting FcεRI expression on the plasmacytoid dendritic cell and the effect of promoting interferon-a production of the plasmacytoid dendritic cell as indicators, and includes at least the following steps:
incubating a test solution containing at least one ingredient selected from the group consisting of a bacterial cell, a bacterial component, and a culture of lactic acid bacteria, and a product obtained by treating any one of them and a plasmacytoid dendritic cell, and incubating a control solution containing a plasmacytoid dendritic cell without the ingredient,
measuring interferon-a production amounts and FcεRI expression levels of the plasmacytoid dendritic cell in the test solution and the control solution after the incubating, and
determining if the ingredient is an active ingredient that activates the plasmacytoid dendritic cell when the measured interferon-a production amount in the test solution is more than the interferon-α production amount in the control solution and the measured FcεRI expression level of the plasmacytoid dendritic cell in the test solution is less than the measured FcεRI expression level of the plasmacytoid dendritic cell in the control solution.

Co-culturing of the test substance with the plasmacytoid dendritic cell, and measurement of the FcεRI expression level in the plasmacytoid dendritic cell and the interferon-α production amount in the plasmacytoid dendritic cell in the method of screening according to one embodiment of the present invention can be performed with reference to the above description.

The method of screening according to one embodiment of the present invention may include various steps and manipulations before, after, or during the above steps as long as it can achieve the objectives of the present invention.

Hereinafter, examples of the present invention will be described in detail, but the present invention is not limited to these examples, and the present invention can adopt various embodiments as long as the object of the present invention can be solved.

### Examples

### [Example 1. Test of Inhibiting FcεRI Expression by Lactic Acid Bacteria]

The effect of inhibiting high-affinity IgE receptor (FcεRI) expression by the lactic acid bacteria was evaluated as follows.

### 1-1. Preparation of Lactic Acid Bacteria Suspension

*Pediococcus acidilactici* strains K15, K226, K531, and JCM8797; *P. pentosaceus* strain NRIC99; *Lactobacillus johnsonii* strain JCM1022; *L. delbrueckii* strain NRIC1688(K1175); *L. pentosus* strains K133 and NRIC1836 were used as lactic acid bacteria.

The strain of "NRIC" indicates that it is a strain distributed from Tokyo University of Agriculture, and the strain of "JCM" is a strain distributed from Institute of Physical and Chemical Research. The strains K15, K226, K531, and K133 were isolated from bran beds, pickles, and wine grapes as lactic acid bacteria-containing substances.

An MRS medium was inoculated with each strain so that a concentration became 1 × 10⁷ cells/ml, and therefore a stock solution of lactic acid bacteria was prepared. The stock solution of lactic acid bacteria was statically cultured at 26°C to 30°C for 24 to 36 hours, and then subjected to a boiling and pasteurization treatment at 95°C for 10 minutes, and therefore a pasteurization-treated culture solution was prepared. The bacterial cells obtained by centrifuging the pasteurization-treated culture solution were washed with a physiological salt solution, and then suspended in the physiological salt solution, and therefore lactic acid bacteria suspension was prepared.

### 1-2. Isolation of Plasmacytoid Dendritic Cell (pDC)

The peripheral blood mononuclear cells were isolated from 50 ml of each peripheral blood of eight non-virus infected healthy subjects A to H by low density gradient centrifugation using a lymphocyte separation solution (NACALAI TESQUE, INC.). Then, CD14-positive cells were removed from the peripheral blood mononuclear cells using CD14 microbeads (Miltenyi Biotec) and MACS (Miltenyi Biotec). After removing the CD14-positive cells, the cells were labeled with FITC-labeled CD304 antibody (Miltenyi Biotec), then reacted with FITC microbeads (Miltenyi Biotec), and CD304-positive cells were isolated by MACS, and these cells (CD14-negative CD304-positive) were used as a pDC.

### 1-3. Co-culturing Lactic Acid Bacteria and pDC

An IMDM medium (GIBCO) containing 10% FBS (Hyclone), 25 mM D-glucose, 25 mM HEPES, and 1 µM sodium pyruvate was used as a cell culture medium. Using a 96-well round-bottom plate (BD Falcon), 1×10⁷ cells of each lactic acid bacteria strain and 2×10⁴ or 4×10⁴ cells of the pDC were co-cultured in each well, and cells and supernatant were recovered after 24 hours. The supernatant recovered from the well in which only pDC was cultured without the lactic acid bacteria was used as a control. Table 1 shows the combination of the subject from which the pDC used was derived and the lactic acid bacteria strain used.

**[Table 1]**

| No. | Lactic acid bacteria strain | Subject |
|---|---|---|
| 1 | *L. johnsonii K1167* | C, D, E, F, G |
| 2 | *L. delbrueckii K1175* | B, D, F, G |
| 3 | *L. pentosus NRIC1836* | F, G |
| 4 | *L. pentosus K133* | F, G |
| 5 | *P. pentosaceus NRIC99* | B, D, F, G, H |
| 6 | *P. acidilactici K226* | F, G |
| 7 | *P. acidilactici K531* | F, G |
| 8 | *P. acidilactici JCM8797* | A, B, C, D, E, F, G, H |
| 9 | *P. acidilactici K15* | A, B, C, D, H |

1-4. Measurement of Activity of Inhibiting FcεRI Expression

The cells recovered after the co-culturing were used to quantify the FcεRI expression using 7AAD (BD Pharmingen) and PE-labeled anti-FcεRIα (Bio Regend) antibodies by flow cytometry (FACS Aria II, Becton Dickinson). With respect to the obtained quantitative value, the control value was set to 1, and the value for each lactic acid bacteria strain was determined by a relative value.

### 1-5. Evaluation

Among these lactic acid bacteria strains tested, *Lactobacillus johnsonii* strain K1167, *Lactobacillus delbrueckii* strain K1175, and *Lactobacillus pentosus* strain NRIC1836 showed higher values (>1) than the control in the pDC derived from one or more subjects. In particular, *Lactobacillus johnsonii* strain K1167 and *Lactobacillus delbrueckii* strain K1175 showed higher values than the control in the pDC derived from two subjects. These lactic acid bacteria strains were judged to have no effect of inhibiting FcεRI expression.

For the remaining lactic acid bacteria strains, the result of calculating the average value among the subjects for each lactic acid bacteria strain from the FcεRI expression level (relative value) of the pDC collected from each subject is shown in FIG. 1. As shown in FIG. 1, the FcεRI expression level of the pDC was as low as 0.9 or less in *Pediococcus acidilactici* and *Pediococcus pentosaceus* with respect to *Lactobacillus pentosus.* In particular, *Pediococcus acidilactici* had a low FcεRI expression level of the pDC in all the strains.

These results indicate that *Pediococcus* lactic acid bacteria have an effect of inhibiting FcεRI expression on pDC, and *Pediococcus acidilactici* have a remarkably excellent effect of inhibiting FcεRI expression on pDC.

### [Example 2. Test of Promoting Interferon-α Production by Lactic Acid Bacteria]

The effect of promoting interferon-α production by the lactic acid bacteria was evaluated as follows.

### 2-1. Measurement of Activity of Promoting Interferon-a Production

Using the culture supernatant recovered after co-culturing with the lactic acid bacteria and the pDC in Example 1, the concentration of IFN-α was quantified using Human IFN-α Matched Antibody Pairs (eBioscience). With respect to the obtained quantitative value, the control value was set to 1, and the value for each lactic acid bacteria strain was determined by a relative value.

### 2-2. Evaluation

Among these lactic acid bacteria strains tested, it was confirmed that the lactic acid bacteria strains other than *Lactobacillus johnsonii* strain K1167 had the ability to induce IFN-α production to the pDC regardless of the subjects from which the pDC was derived and the type of lactic acid bacteria strains. As *Lactobacillus johnsonii* strain K1167, the pDC derived from any of the subjects produced less IFN-α (1.8 on average among the subjects) and showed only a slight increase over the control.

On the other hand, among the lactic acid bacteria strains tested, *Lactobacillus delbrueckii* strain K1175 had the greatest ability to induce IFN-α production to the pDC. In the viewpoint of the combination with the results of Example 1, which showed that *Lactobacillus delbrueckii* strain K1175 did not have the effect of inhibiting FcεRI expression, it was found that the effect of promoting interferon-a production to the pDC and the effect of inhibiting FcεRI expression were not necessarily correlated.

These results show that *Pediococcus* lactic acid bacteria such as *Pediococcus acidilactici* and *Pediococcus pentosaceus* have the effect of inhibiting FcεRI expression to the pDC and also have the effect of promoting interferon-a production, which is a very surprising phenomenon that was first discovered by the present inventors.

### [Example 3. Comparative Test with TLR Ligand for Effect of Inhibiting FcεRI Expression]

Comparison of the activity of inhibiting FcεRI expression by *Pediococcus acidilactici* K15 strain and the activity by TLR ligand was evaluated as follows.

### 3-1. Measurement of Inhibiting FcεRI Expression by TLR Ligand

As the pDC collected from the subject H, co-culturing with the pDC was performed as in Example 1, except that instead of each lactic acid bacteria strain, Imiquimod (R837; invivogen) which is a ligand of TLR7, ssRNA40/LyoVec (invivogen) which is a ligand of TLR8, and ODN2216 (CpG; invivogen) which is a ligand of TLR9, were used at a final concentration of 10 µg/ml. A supernatant recovered from wells in which only the pDC was cultured without using K15 strain or the ligand was used as a control.

Using the cells recovered after the co-culturing, RNA was extracted by NucleoSpin RNA XS (Macherey-Nagel), and the mRNA expression levels of FcεRIα and β-actin were measured by quantitative PCR using PrimeScript RT Reagent Kit (Takara Bio) and TB Green Premix Ex Taq II (Takara Bio). Each primer was purchased from Takara Bio Inc. The FcεRIα mRNA expression level was standardized using β-actin measured value, and the value of the control (Med) was set to 1, and the value of K15 strain or ligand was determined by relative value.

### 3-2. Evaluation

The relative amounts of FcεRI mRNA in each TLR ligand and K15 strain are shown in FIG.2. As shown in FIG.2, according to the relative amount of FcεRI mRNA, it was found that the activity of inhibitin FcεRI expression in K15 strain was as strong as or stronger than the activity of each TLR ligand.

### Industrial Applicability

The composition according to one embodiment of the present invention contains the active ingredient applicable to both oral and parenteral forms, are useful for an intake individual expecting antiviral activity, anti-infective activity, anti-allergy activity, anti-hay fever activity, and anti-asthma activity through the effect of inhibiting FcεRI expression on the pDC, and can be used as foods and drinks, medicines, quasi-drugs, cosmetics, and the like which contribute to the health and welfare of such an intake individual.

### Cross-Reference to Related Application

The present application claims the benefit of priority to Japanese Patent Application No. 2018-150152, filed on August 9, 2018, the disclosure of which is incorporated herein by reference in its entirety.

## Claims

1. A composition for activating plasmacytoid dendritic cell,
comprising at least one active ingredient selected from the group consisting of a bacterial cell, a bacterial component, and a culture of *Pediococcus* lactic acid bacteria, and a product obtained by treating any one of them, and
having an effect of inhibiting FcεRI expression on a plasmacytoid dendritic cell.

2. The composition according to claim 1,
wherein the composition is a composition having an effect of promoting interferon-α production on a plasmacytoid dendritic cell.

3. The composition according to claim 1 or 2,
wherein *Pediococcus* lactic acid bacteria is at least one *Pediococcus* lactic acid bacteria selected from the group consisting of *Pediococcus acidilactici* and *P. pentosaceus.*

4. The composition according to any one of claims 1 to 3,
wherein the composition has a stronger effect of inhibiting FcεRI expression on a plasmacytoid dendritic cell than that when using *Lactobacillus pentosus* strain K133 in the same amount as the active ingredient.

5. The composition according to any one of claims 1 to 4,
wherein the composition is at least one anti-desease composition selected from the group consisting of an antiviral composition, a composition for anti-infectious diseases, a composition for anti-allergy, a composition for anti-hay fever, and a composition for anti-asthma.

6. A composition for foods and drinks which comprises the composition according to any one of claims 1 to 5.

7. A method of screening lactic acid bacteria for activating plasmacytoid dendritic cell, comprising:
incubating a test solution comprising at least one ingredient selected from the group consisting of a bacterial cell, a bacterial component, and a culture of lactic acid bacteria, and a product obtained by treating any one of them and a plasmacytoid dendritic cell, and incubating a control solution comprising a plasmacytoid dendritic cell without the ingredient,
measuring FcεRI expression levels of the plasmacytoid dendritic cell in the test solution and the control solution after the incubating, and
determining if the ingredient is an active ingredient that activates the plasmacytoid dendritic cell when the measured FcεRI expression level of the plasmacytoid dendritic cell in the test solution is less than the measured FcεRI expression level of the plasmacytoid dendritic cell in the control solution.

8. A method of screening lactic acid bacteria for activating plasmacytoid dendritic cell, comprising:
incubating a test solution comprising at least one ingredient selected from the group consisting of a bacterial cell, a bacterial component, and a culture of lactic acid bacteria, and a product obtained by treating any one of them and a plasmacytoid dendritic cell, and incubating a control solution comprising a plasmacytoid dendritic cell without the ingredient,
measuring interferon-α production amounts and FcεRI expression levels of the plasmacytoid dendritic cell in the test solution and the control solution after the incubating, and
determining if the ingredient is an active ingredient that activates the plasmacytoid dendritic cell when the measured interferon-a production amount in the test solution is more than the interferon-a production amount in the control solution and the measured FcεRI expression level of the plasmacytoid dendritic cell in the test solution is less than the measured FcεRI expression level of the plasmacytoid dendritic cell in the control solution.

9. A method of inhibiting FcεRI expression on plasmacytoid dendritic cell, comprising:
using at least one active ingredient selected from the group consisting of a bacterial cell, a bacterial component, and a culture of *Pediococcus* lactic acid bacteria, and a product obtained by treating any one of them.
